# EUROPÄISCHE PATENTSCHRIFT

(11) **EP 1 572 110 B1**
(45) Veröffentlichungstag und Bekanntmachung des Hinweises auf die Patenterteilung: **09.09.2009**
(21) Anmeldenummer: 03789178.5
(22) Anmeldetag: 09.12.2003
(51) Int. Cl.: A61K 6/00, A61K 6/02, A61K 6/10, A61K 6/06, A61K 6/08

(54) **DENTALMATERIAL MIT BAKTERIOSTATISCHEN UND/ODER BAKTERIZIDEN SUBSTANZEN**
DENTAL MATERIAL CONTAINING BACTERIOSTATIC AND/OR BACTERICIDAL SUBSTANCES
MATERIAU DENTAIRE CONTENANT DES SUBSTANCES BACTERIOSTATIQUES ET/OU BACTERICIDES

(30) Priorität: 20.12.2002 DE 10261241
(43) Veröffentlichungstag der Anmeldung: 14.09.2005
(73) Patentinhaber: 3M ESPE AG, 82229 Seefeld (DE)
(72) Erfinder: HAEBERLEIN, Ingo, 82362 Weilheim (DE); RICHTER, Bettina, 82205 Gilching (DE)
(86) Internationale Anmeldenummer: PCT/EP2003/013915
(87) Internationale Veröffentlichungsnummer: WO 2004/058193

(56) Entgegenhaltungen:
- EP-A- 0 147 021
- WO-A-90/06138
- WO-A-94/03174
- DE-A- 2 628 265
- DE-A- 19 937 092
- GORMAN S P ET AL: "A COMPARATIVE STUDY OF THE MICROBIAL ANTI-ADHERENCE CAPACITIES OF THREE ANTIMICROBIAL AGENTS" JOURNAL OF CLINICAL PHARMACY AND THERAPEUTICS, BLACKWELL SCIENTIFIC PUBLICATION, OXFORD, GB, Bd. 12, 1987, Seiten 393-399, XP002909130 ISSN: 0269-4727
- REYNOLDS ET AT: "Taurolin as an oral rinse I. Antimicrobial effects in vitro and in vivo" CLINICAL PREVENTIVE DENTISTRY, Bd. 13, Nr. 2, - 1991 Seiten 13-22, XP009029748

## Beschreibung

Die Erfindung betrifft ein Dentalmaterial mit bakteriostatischen und/oder bakteriziden Substanzen. Ferner betrifft die Erfindung die Verwendung von bakteriostatischen und/oder bakteriziden Substanzen zur Herstellung von Dentalmaterialien.

Dentalmaterialien mit unterschiedlichen Wirkstoffzusätzen, die antimikrobielle bzw. bakteriostatische und/oder bakterizide Eigenschaften aufweisen, sind bekannt.

So wird in der EP 0 674 896 B1 eine Dentalmasse aus Kunstharzen beschrieben, die eine Quarternär-Ammoniumverbindung enthält, die keimtötende und bakteriostatische Wirkung hat und ein Gemisch von ätherischen Ölen mit Minzöl, Eukalyptusöl und Bergamotöl enthält. Nachteilig an dieser Dentalmasse ist der charakteristische Geruch, der auf den Zusatz der ätherischen Öle zurückzuführen ist. Weiterhin wird der Einsatz von quartären Ammoniumverbindungen aufgrund der mit diesen Substanzen verbundenen unerwünschten Wirkungen in Dentalmassen und der Tatsache, dass diese Materialien zum dauerhaften Verbleib im Mund des Patienten gedacht sind, von Fachleuten teilweise abgelehnt.

Weiterhin ist die Verwendung der antimikrobiell wirksamen Substanzen Taurolidin sowie deren Metabolit Taurultam gegen Parodontose in Form einer Spüllösung aus der DE 26 28 265 C2 bekannt.

In der WO 98/48766 ist ein Dentalmaterial offenbart, das als antimikrobielle Substanz 2,4,4'-Trichloro-2'-hydroxydiphenylether (Triclosan) enthält. Die Verwendung dieser Substanz als Zusatz in Dentalmaterial bewirkt eine zeitlich begrenzte antimikrobielle Wirksamkeit, da das Triclosan in Abhängigkeit von Anfangskonzentration und Speichelfluss aus dem Material herausgelöst und abtransportiert wird.

Es ist daher eine Aufgabe der vorliegenden Erfindung, ein Dentalmaterial zur Verfügung zu stellen, bei dem die oben beschriebenen Nachteile der bisher bekannten Materialien vermieden werden können.

Erfindungsgemäß wird diese Aufgabe gelöst durch ein Dentalmaterial enthaltend mindestens eine Substanz, deren bakteriostatische und/(oder bakterizide Wirsamkeit in Gegenwart intraoraler Mikroorganismen ausgebildet wird.

Der Einsatz einer derartigen Substanz in einem Dentalmaterial führt zu einer orts- und zeitspezifischen bakteriostatischen und/oder bakteriziden Wirkung durch die Freisetzung eines Wirstoffs. Dies bedeutet, dass durch die Substanz der Wirstoff zunächst in einer inaktiven Form in dem Dentalmaterial vorgehalten wird. Die Herstellung, Verpackung, Versand sowie die Lagerung des Dentalmaterials erfolgt mit der Substanz. Der Wirstoff liegt somit in einer inaktiven Form vor. Auch bei der Verarbeitung des Dentalmaterials kann der Wirstoff in dieser Form vorliegen.

Zusätzlich kann die in dem Dentalmaterial enthaltene Substanz auch eine initiale bakteriostatische und/oder bakterizide Wirkung aufweisen, die sich beispielsweise gleich bei der Verarbeitung entfaltet.

Erst bei einer aufgrund äußerer Umstände gegebenen Notwendigkeit, wie beispielweise der Besiedelung der Mundhöhle des Patienten mit pathogenen Mikroorganismen, bildet die Substanz ihre bakteriostatische und/oder bakterizide Wirkung aus, indem sie den Wirstoff freisetzt. Das gleiche gilt auch, wenn das Dentalmaterial während der Verarbeitung am Patienten trotz Desinfektions- und Sterilisationsmaßnahmen mit Mikroorganismen z.B. am Verarbeitungswerkzeug oder am Handschuh des Zahnarztes in Berührung kommt.

Besonders vorteilhaft ist hierbei, dass das Dentalmaterial, das die den Wirkstoff freisetzende Substanz enthält, den Patienten nicht beeinflusst. Erst bei Ausbildung der Wirksamkeit tritt die bakteriostatische und/oder bakterizide Wirkung des Wirkstoffs ein. Die Ausbildung der Wirksamkeit ist dabei abhängig von der Gegenwart intraoraler Mikroorganismen. Hierbei ist sie insbesondere abhängig von der Gegenwart pathogener und/oder in der Mundflora des Patienten unerwünschter Mikroorganismen.

Es wurde festgestellt, dass aufgrund der orts- und zeitspezifischen Wirkung das erfindungsgemäße Dentalmaterial seine bakteriostatischen und/oder bakteriziden Eigenschaften nur an den Stellen entwickelt, an denen aufgrund der Gegenwart intraoraler Mikroorganismen ein Bedarf an einer bakteriostatischen und/oder bakteriziden Wirkung besteht. Der Patient wird somit nur an den Stellen und nur in dem Umfang mit einem Wirkstoff belastet, wie es aufgrund des Auftretens von intraoralen Mikroorganismen notwendig ist. Dies ist beispielsweise der Fall in dem ca. 20 µm breiten Spalt zwischen einer Zahnrestauration und des gesunden Zahnteils. Dieser sogenannte Randspalt kann zwischen einer Zahnfüllung, einem Inlay, einem Onlay, einer Krone oder Brücke bzw. der aus Zahnzement oder Bonding bestehenden Haftvermittlerschicht und der gesunden Zahnsubstanz auftreten. Er bildet sich beispielsweise bei der Aushärtung einer Kunststofffüllung durch den polymerisationsbedingten Schrumpf. Dieser Randspalt wird in einem gewissen zeitlichen Abstand nach einer Zahnbehandlung oft mit intraoralen Mikroorganismen besiedelt.

Ferner bildet sich der Wirkstoff erst im Bedarfsfall. Dies hat zur Folgte, dass die Wirkstoffkorizentration nicht anfänglich sehr hoch ist und zeitabhängig nachlässt, sondern dass die Konzentration des aktiven Wirkstoffs dann am größten ist, wenn aktuell aufgrund der Gegenwart und Vermehrung intraoraler Mikroorganismen der Bedarf entsteht. Der Patient wird somit nicht einer dauerhaften Grundbelastung mit einem Wirkstoff ausgesetzt.

Dabei beruht die Ausbildung der Wirksamkeit beispielsweise auf einer Modifikation der Substanz, die durch enzymatische, physikalische, chemische oder biochemische Milieuänderung bewirkt wird. Eine solche Modifikation kann eine Änderung des Mundmilieus durch Enzymsekretion der Mikroorganismen sein. Auch durch Lyse und die damit verbundene Freisetzung von bestimmten Substanzen, wie z.B. Enzymen, Metaboliten kann sich eine Milieuänderung ergeben. Ferner können die in der Zellwand, der Plasmamembran oder im periplasmatischen Raum der Mikroorganismen vorhandenen Enzyme auch unabhängig von einer Sekretion chemische Veränderungen oder Konzentrationsveränderungen im Mundmilieu bewirken.

Denkbar ist auch eine Veränderung der physikalischen oder chemischen Milieubedingungen, wie zum Beispiel des pH-Werts, der Salzkonzentration, der Temperatur oder ähnliches. Hierdurch wird eine Modifikation der Substanz durch beispielsweise Hydrolyse, Umesterung, Veränderung der Konfiguration erreicht.

Besonders vorteilhaft an dem erfindungsgemäßen Dentalmaterial ist, dass die Substanz in dem Bereich zwischen Dentin oder Schmelz und Dentalmaterial angereichert und/oder vorgehalten werden kann. Wie bereits beschrieben, können sich beispielsweise bei einer Füllung im Randspalt Mikroorganismen ansiedeln. Genau in diesen Fällen ist die Anwesenheit eines bakteriostatischen und/oder bakteriziden Wirkstoffs in ausreichender Konzentration gewünscht. Bei dem erfindungsgemäßen Dentalmaterial kann dies insbesondere aufgrund einer Anreicherung durch die Diffusion des Wirkstoffs in den Bereich zwischen Dentin oder Schmelz und Dentalmaterial erreicht werden. Dabei ist es durch die zeit- und ortsspezifische Ausbildung des Wirkstoffs aus der Substanz gewährleistet, dass der Wirkstoff in ausreichender Konzentration vorhanden ist, um einen Diffusionsgradienten zu erreichen.

Zur Verhinderung der Diffusion der den Wirkstoff freisetzenden Substanz aus dem Dentalmaterial heraus kann die Substanz entsprechend derivatisiert werden. Ferner kann sie kovalent in das Dentalmaterial eingebunden werden. Durch diese Maßnahmen kann die Substanz in dem Bereich zwischen Dentin oder Schmelz und Dentalmaterial an der Oberfläche des Dentalmaterials vorgehalten werden und orts- und zeitspezifisch aufgrund der enzymatischen, physikalischen, chemischen oder biochemischen Milieuänderung, ausgelöst durch die intraoralen Mikroorganismen, freigesetzt werden.

Dabei ist es möglich, dass die orts- und zeitspezifische Freisetzung der Substanz aus dem Dentalmaterial und die Ausbildung der Wirksamkeit durch gleichartige oder unterschiedliche enzymatische, physikalischen, chemische oder biochemische Milieuänderung, ausgelöst durch die intraoralen Mikroorganismen, bewirkt werden.

Besondern vorteilhaft ist es, wenn die Freisetzung der Substanz aus dem Dentalmaterial aufgrund enzymatischer Abspaltung erfolgt.

Ferner kann die Substanz aufgrund einer Derivatisierung an der Diffusion aus dem Dentalmaterial gehindert werden oder in das Dentalmaterial kovalent eingebunden werden und in dem Bereich zwischen Dentin oder Schmelz und Dentalmaterial an der Oberfläche des Dentalmaterials vorgehalten werden ohne dass eine Freisetzung der Substanz erfolgt. Dabei kann die Ausbildung der Wirksamkeit auf einer Modifikation der Substanz beruhen, die durch enzymatische, physikalische, chemische oder biochemische Milieuänderung, ausgelöst durch die intraoralen Mikroorganismen, bewirkt wird, wobei eine Trennung vom Dentalmaterial nicht stattfindet.

Bei einem derartigen Dentalmaterial kann die Ausbildung der Wirksamkeit in mehreren Schritten erfolgen. Dies können beispielsweise gleichartige oder unterschiedliche enzymatische, physikalische, chemische oder biochemische Milieuänderungen, ausgelöst durch die intraoralen Mikroorganismen, sein.

Ferner kann die Substanz auch nach der Modifikation und der damit verbundenen Ausbildung der Wirksamkeit durch Derivatisierung an der Diffusion aus dem Dentalmaterial gehindert bleiben oder in das Dentalmaterial kovalent eingebunden bleiben.

### Beispiele für verwendbare Substanzen/Materialien:

### Herstellung von Taurolidin:

In der DE 195 15 976 C1 wird die Verwendung von β-Azidoethansulfonylazid zur Herstellung von Taurinamid bzw. zur Herstellung von Taurolidin beschrieben. Ferner ist in DE 197 08 782 C1 ein Verfahren zur Herstellung von 2-Aminoethansulfonylazidsäureadditionssalzen beschrieben, die dann in bekannter Weise zu Taurolidin oder Taurultam umgesetzt werden können.

### Beispiel 1:

### Abformmaterial auf Silikon-Basis

Zu dem kommerziell erhältlichen Standard-Silikonabformaterial Dimension Penta H (Fa. ESPE Dental AG, Seefeld, Deutschland) wurde Taurolidin gegeben, in dem sowohl in die Katalysatorpaste als auch in die Basispaste Taurolidin bis zu einem Endgehalt von 2,5 % eingeknetet wurde. Durch den Taurolidin-Zusatz wurde weder das Abbindeverhalten noch die Lagerstabilität des Silikonabformmaterials beeinflußt. Kreisrunde Probenkörper mit und ohne Taurolidin-Zusatz wurden hergestellt und jeweils mit einem Tropfen einer Lactobacillus paracasii Kulturlösung versehen. Mit dem kommerziell bei Molecular Probes, Leiden, Niederlande erhältlichen Kit zur Lebend/Tod-Bestimmung von Bakterien (LIVE/DEAD BacLight Bacterial Viability Kit) wurde die bakteriozide Wirkung des in der Silikonabformmasse befindlichen Taurolidin unter dem Fluoreszenzmikroskop (Axioplan 2, Fa. Zeiss) evaluiert. Die Silikonabformmasse mit Taurolidin zeigte ca. 90 % weniger lebende Bakterien als die Silikonabformmase ohne Taurolidin-Zusatz.

### Beispiel 2:

### Abformmaterial auf Polyether-Basis

Zu dem kommerziell erhältlichen Standard-Polyetherabformaterial Impregum Penta (Fa. ESPE Dental AG, Seefeld, Deutschland) wurde Taurolidin gegeben, in dem sowohl in die Katalysatorpaste als auch in die Basispaste Taurolidin bis zu einem Endgehalt von 2,5 % eingeknetet wurde. Durch den Taurolidin-Zusatz wurde das Abbindeverhalten nicht beeinflußt. Kreisrunde Probenkörper mit und ohne Taurolidin-Zusatz wurden hergestellt und jeweils mit einem Tropfen einer Lactobacillus paracasii Kulturlösung versehen. Mit dem kommerziell bei Molecular Probes, Leiden, Niederlande erhältlichen Kit zur Lebend/Tod-Bestimmung von Bakterien (LIVE/DEAD BacLight Bacterial Viability Kit) wurde die bakteriozide Wirkung des in der Silikonabformmasse befindlichen Taurolidin unter dem Fluoreszenzmikroskop (Axioplan 2, Fa. Zeiss) evaluiert. Die Polyetherabformmasse mit Taurolidin zeigte ca. 90 % weniger lebende Bakterien als die Polyehterabformmase ohne Taurolidin-Zusatz.

### Beispiel 3:

### Abformmaterial auf Alginatbasis

Zu dem kommerziell erhältlichen Standard-Alginat Palgat Quick (Fa. ESPE Dental AG, Seefeld, Deutschland) wurde Taurolidin bis zu einem Endgehalt von 2 % gegeben. Durch den Taurolidin-Zusatz wurde das Abbindeverhalten des Alginats nicht beeinflußt. Kreisrunde Probenkörper mit und ohne Taurolidin-Zusatz wurden hergestellt und jeweils mit einem Tropfen einer Lactobacillus paracasii Kulturlösung versehen. Mit dem kommerziell bei Molecular Probes, Leiden, Niederlande erhältlichen Kit zur Lebend/Tod-Bestimmung von Bakterien (LIVE/DEAD BacLight Bacterial Viability Kit) wurde die bakteriozide Wirkung des in der Alginatmasse befindlichen Taurolidins unter dem Fluoreszenzmikroskop (Axioplan 2, Fa. Zeiss) evaluiert. Die Alginatabformmasse mit Taurolidin zeigte zw. 90 % und 95 % weniger lebende Bakterien als die Alginatabformmase ohne Taurolidin-Zusatz.

### Beispiel 4:

### Füllungsmaterial auf Composit-Basis

In das kommerziell erhältliche Composit-Füllungsmaterial Pertac II (Fa. ESPE Dental AG, Seefeld, Deutschland) wurde Taurolidin (< 42 µm) bis zu einem Endgehalt von 2,5 % eingeknetet. Durch den Taurolidin-Zusatz wurden die physikalischen Eigenschaften von Pertac II nur geringfügig beeinflußt. Kreisrunde Probenkörper mit und ohne Taurolidin-Zusatz wurden hergestellt und jeweils mit einem Tropfen einer Lactobacillus paracasii Kulturlösung versehen. Mit dem kommerziell bei Molecular Probes, Leiden, Niederlande erhältlichen Kit zur Lebend/Tod-Bestimmung von Bakterien (LIVE/DEAD BacLight Bacterial Viability Kit) wurde die bakteriozide Wirkung des im Glasionomerzement befindlichen Taurolidins unter dem Fluoreszenzmikroskop (Axioplan 2, Fa. Zeiss) evaluiert. Der Composit mit Taurolidin zeigte nahezu keine lebende Bakterien im Vergleich zu dem Composit ohne Taurolidin-Zusatz.

### Beispiel 5:

### Füllungsmaterial auf Compomer-Basis

In das kommerziell erhältliche Standard-Compomer Hytac II (Fa. ESPE Dental AG, Seefeld, Deutschland) wurde Taurolidin (< 42 µm) bis zu einem Endgehalt von 2,5 % eingebracht. Durch den Taurolidin-Zusatz wurden die physikalischen Eigenschaften von Hytac nur geringfügig beeinflußt. Kreisrunde Probenkörper mit und ohne Taurolidin-Zusatz wurden hergestellt und jeweils mit einem Tropfen einer Lactobacillus paracasii Kulturlösung versehen. Mit dem kommerziell bei Molecular Probes, Leiden, Niederlande erhältlichen Kit zur Lebend/Tod-Bestimmung von Bakterien (LIVE/DEAD BacLight Bacterial Viability Kit) wurde die bakteriozide Wirkung des im Glasionomerzement befindlichen Taurolidins unter dem Fluoreszenzmikroskop (Axioplan 2, Fa. Zeiss) evaluiert. Der Compomer mit Taurolidin zeigte 95 % weniger lebende Bakterien im Vergleich zu dem Compomer ohne Taurolidin-Zusatz.

### Beispiel 6:

### Füllungsmaterial auf Glasionomerzementbasis

Zu dem kommerziell erhältlichen Standard-Glasionomerzement Ketac Molar (Fa. ESPE Dental AG, Seefeld, Deutschland) wurde zur pulvrigen Komponente gesiebtes Taurolidin (< 42 µm) bis zu einem Endgehalt von 0,8 % gegeben. Durch den Taurolidin-Zusatz wurden die physikalischen Eigenschaften von Ketac Molar nur geringfügig beeinflußt. Kreisrunde Probenkörper mit und ohne Taurolidin-Zusatz wurden hergestellt und jeweils mit einem Tropfen einer Lactobacillus paracasii Kulturlösung versehen. Mit dem kommerziell bei Molecular Probes, Leiden, Niederlande erhältlichen Kit zur Lebend/Tod-Bestimmung von Bakterien (LIVE/DEAD BacLight Bacterial Viability Kit) wurde die bakteriozide Wirkung des im Glasionomerzement befindlichen Taurolidins unter dem Fluoreszenzmikroskop (Axioplan 2, Fa. Zeiss) evaluiert. Der Glasionomerzement mit Taurolidin zeigte ca. 85 % weniger lebende Bakterien als der Glasionomerzement ohne Taurolidin-Zusatz.

### Beispiel 7:

### Provisorische Füllungsmaterialien

Zu dem kommerziell erhältlichen Standardfüllungsmaterial zur provisorischen Kavitätenversorgung Cavit LC (Fa. ESPE Dental AG, Seefeld, Deutschland) wurde gesiebtes Taurolidin (< 42 µm) bis zu einem Endgehalt von 2,5 % zugegegben und anschließend bis zur Homogenität geknetet. Durch den Taurolidin-Zusatz wurden die physikalischen Eigenschaften von Cavit LC nur geringfügig beeinflußt. Kreisrunde Probenkörper mit und ohne Taurolidin-Zusatz wurden hergestellt und jeweils mit einem Tropfen einer Lactobacillus paracasii Kulturlösung versehen. Mit dem kommerziell bei Molecular Probes, Leiden, Niederlande erhältlichen Kit zur Lebend/Tod-Bestimmung von Bakterien (LIVE/DEAD BacLight Bacterial Viability Kit) wurde die bakteriozide Wirkung des im Cavit LC befindlichen Taurolidins unter dem Fluoreszenzmikroskop (Axioplan 2, Fa. Zeiss) evaluiert. Cavit LC mit Taurolidin zeigte z.T. keinerlei lebende Bakterien.

### Beispiel 8:

### Glasionomerbefestigungszemente

Zu dem kommerziell erhältlichen Standard-Glasionomerbefestigungszement Ketac Cem (Fa. ESPE Dental AG, Seefeld, Deutschland) wurden zur pulverigen Komponente gesiebtes Taurolidin (< 42 µm) bis zu einem Endgehalt von 2,5 % zugegeben und anschließend bis zur Homogenität gemischt. Durch den Taurolidin-Zusatz wurden die physikalischen Eigenschaften von Ketac Cem nur geringfügig beeinflußt. Kreisrunde Probenkörper mit und ohne Taurolidin-Zusatz wurden hergestellt und jeweils mit einem Tropfen einer Lactobacillus paracasii Kulturlösung versehen. Mit dem kommerziell bei Molecular Probes, Leiden, Niederlande erhältlichen Kit zur Lebend/Tod-Bestimmung von Bakterien (LIVE/DEAD BacLight Bacterial Viability Kit) wurde die bakteriozide Wirkung des im Cavit befindlichen Taurolidins unter dem Fluoreszenzmikroskop (Axioplan 2, Fa. Zeiss) evaluiert. Der Glasionomerbefestigungszement mit Taurolidin zeigte z.T. keinerlei lebende Bakterien.

### Beispiel 9 (nicht erfindungsgemäß)

### Bondingmaterial

In das kommerziell erhältliche Standard-Bonding Visio-Bond (Fa. ESPE Dental AG, Seefeld, Deutschland) wurde Taurolidin bis zu einem Endgehalt von 2,0 % eingebracht. Durch den Taurolidin-Zusatz wurden die physikalischen Eigenschaften von Visio-Bond nur geringfügig beeinflußt. Kreisrunde Probenkörper mit und ohne Taurolidin-Zusatz wurden durch Belichten von 400 µl Visio-Bond in einer 24er Mikrotiterplatte nach Gebrauchsanweisung hergestellt. Die ausgehärteten Plättchen wurden entnommen und jeweils mit einem Tropfen einer Lactobacillus paracasii Kulturlösung versehen. Mit dem kommerziell bei Molecular Probes, Leiden, Niederlande erhältlichen Kit zur Lebend/Tod-Bestimmung von Bakterien (LIVE/DEAD BacLight Bacterial Viability Kit) wurde die bakteriozide Wirkung des im Glasionomerzement befindlichen Taurolidins unter dem Fluoreszenzmikroskop (Axioplan 2, Fa. Zeiss) evaluiert. Die Bondingproben mit Taurolidin zeigten 90 % weniger lebende Bakterien im Vergleich zu den Bondingsproben ohne Taurolidin-Zusatz.

## Patentansprüche

1. Dentalmaterial enthaltend mindestens eine Substanz, deren bakteriostatische und/oder bakterizide Wirksamkeit in Gegenwart intraoraler Mikroorganismen ausgebildet wird nämlich Taurolidin, wobei das Dentalmaterial gewählt ist aus einer dentalen Abformmasse, einem dentalen Füllungsmaterial, einem Glasionomerzement oder einem dentalen provisorischen Füllungsmaterial.

2. Dentalmaterial nach Anspruch 1, **dadurch gekennzeichnet, dass** die Ausbildung der Wirksamkeit auf einer Modifikation der Substanz beruht, die durch eine enzymatische, physikalische, chemische oder biochemische Milieuänderung, ausgelöst durch die intraoralen Mikroorganismen, bewirkt wird.

3. Dentalmaterial nach einem der Ansprüche 1 oder 2, **dadurch gekennzeichnet, dass** die Substanz in dem Bereich zwischen Dentin oder Schmelz und Dentalmaterial angereichert und/oder vorgehalten wird.

4. Dentalmaterial nach einem der Ansprüche 1 bis 3, **dadurch gekennzeichnet, dass** sich die Substanz durch Diffusion in den Bereich zwischen Dentin oder Schmelz und Dentalmaterial anreichert.

5. Dentalmaterial nach einem der Ansprüche 1 bis 3, **dadurch gekennzeichnet, dass** die Substanz durch Derivatisierung an der Diffusion aus dem Dentalmaterial gehindert ist oder in das Dentalmaterial kovalent eingebunden ist und in dem Bereich zwischen Dentin oder Schmelz und Dentalmaterial an der Oberfläche des Dentalmaterials vorgehalten wird und dass die Substanz orts- und zeitspezifisch aufgrund der enzymatischen, physikalischen, chemischen oder biochemischen Milieuänderung, ausgelöst durch die intraoralen Mikroorganismen, freigesetzt wird.

6. Dentalmaterial nach Anspruch 5, **dadurch gekennzeichnet, dass** die orts- und zeitspezifische Freisetzung der Substanz und die Ausbildung der Wirksamkeit durch gleichartige oder unterschiedliche enzymatische, physikalische, chemische oder biochemische Milieuänderung, ausgelöst durch die intraoralen Mikroorganismen, bewirkt werden.

7. Dentalmaterial nach einem der Ansprüche 1 bis 6, **dadurch gekennzeichnet, dass** die Freisetzung der Substanz aufgrund enzymatischer Abspaltung erfolgt.

8. Dentalmaterial nach einem der Ansprüche 1 bis 4, **dadurch gekennzeichnet, dass** die Substanz durch Derivatisierung an der Diffusion aus dem Dentalmaterial gehindert ist oder in das Dentalmaterial kovalent eingebunden ist und in dem Bereich zwischen Dentin oder Schmelz und Dentalmaterial an der Oberfläche des Dentalmaterials vorgehalten wird und die Ausbildung der Wirksamkeit auf einer Modifikation des Wirkstoffs beruht, die durch enzymatische, physikalische, chemische oder biochemische Milieuänderung, ausgelöst durch die intraoralen Mikroorganismen, bewirkt wird, wobei keine Freisetzung der Substanz erfolgt.

9. Dentalmaterial nach Anspruch 8, **dadurch gekennzeichnet, dass** die Ausbildung der Wirksamkeit in mehreren Schritten durch gleichartige oder unterschiedliche enzymatische, physikalische, chemische oder biochemische Milieuänderung, ausgelöst durch die intraoralen Mikroorganismen, bewirkt wird.

10. Dentalmaterial nach Anspruch 8 oder 9, **dadurch gekennzeichnet, dass** die Substanz nach Ausbildung der Wirksamkeit durch Derivatisierung an der Diffusion aus dem Dentalmaterial gehindert bleibt oder in das Dentalmaterial kovalent eingebunden bleibt.

11. Dentalmaterial nach einem der Ansprüche 1 bis 10, enthaltend
(a) 0,01 - 10 % einer Substanz, deren bakteriostatische und/oder bakterizide Wirksamkeit in Gegenwart intraoraler Mikroorganismen ausgebildet wird,
(b) 3 - 80% einer polymerisierbaren Verbindung
(c) 0,01 - 25 % übliche Initiatoren und/oder Beschleuniger und/oder Verzögerer
(d) 0 - 50 % übliche Hilfsstoffe
(e) 0 - 90 % übliche Füllstoffe.

12. Dentalmaterial nach einem der Ansprüche 1 bis 11, enthaltend
(a) 0,1 - 5 % einer Substanz, deren bakteriostatische und/oder bakterizide Wirksamkeit in Gegenwart intraoraler Mikroorganismen ausgebildet wird,
(b) 3 - 80 % einer polymerisierbaren Verbindung
(c) 0,01 - 25 % übliche Initiatoren und/oder Beschleuniger und/oder Verzögerer
(d) 0 - 50 % übliche Hilfsstoffe
(e) 0 - 90 % übliche Füllstoffe.

13. Dentalmaterial nach einem der Ansprüche 1 bis 12, enthaltend
(a) 0,1 - 3 % einer Substanz, deren bakteriostatische und/oder bakterizide Wirksamkeit in Gegenwart intraoraler Mikroorganismen ausgebildet wird,
(b) 3 - 80 % einer polymerisierbaren Verbindung
(c) 0,01 - 25 % übliche Initiatoren und/oder Beschleuniger und/oder Verzögerer
(d) 0 - 50 % übliche Hilfsstoffe
(e) 0 - 90 % übliche Füllstoffe.

14. Verwendung einer Substanz, deren bakteriostatische und/oder bakterizide Wirksamkeit in Gegenwart intraoraler Mikroorganismen ausgebildet wird, zur Herstellung eines Dentalmaterials, wobei es sich bei dieser Substanz um Taurolidin handelt und die Dentalmaterialien gewählt sind aus einer dentalen Abformmasse, einem dentalen Füllungsmaterial, einem Glasionomerzement oder einem dentalen provisorischen Füllungmaterial.

15. Verwendung einer Substanz, deren bakteriostatische und/oder bakterizide Wirksamkeit in Gegenwart intraoraler Mikroorganismen ausgebildet wird zur Herstellung einer dentalen Abformmasse, eines dentalen Füllungsmaterials, eines Glasionomerzements, oder eines dentalen provisorischen Füllungsmaterials wobei es sich bei dieser Substanz um Taurolidin handelt.

## Claims

1. Dental material comprising at least one substance whose bacteriostatic and/or bactericidal activity develops in the presence of intraoral microorganism, viz. taurolidine, wherein the dental material is selected from a dental moulding material, a dental filling material, a glass ionomer cement or a dental provisional filling material.

2. Dental material according to Claim 1, **characterized in that** the development of activity is based on the substance being modified by an enzymatic, physical, chemical or biochemical change in the surroundings which is induced by the intraoral microorganisms.

3. Dental material according to either of Claims 1 and 2, **characterized in that** the substance is accumulated and/or stored in the region between dentine or enamel and dental material.

4. Dental material according to any one of Claims 1 to 3, **characterized in that** the substance accumulates in the region between dentine or enamel and dental material by diffusion.

5. Dental material according to any one of Claims 1 to 3, **characterized in that** the substance is prevented from diffusing out of the dental material by derivatization, or is covalently bonded in the dental material and is stored in the region between dentine or enamel and dental material at the surface of the dental material, and **in that** the substance is released location- and time-specifically owing to the enzymatic, physical, chemical or biochemical change in the surroundings which is induced by the intraoral microorganisms.

6. Dental material according to Claim 5, **characterized in that** the location- and time-specific release of the substance and the development of activity are brought about by a similar or different enzymatic, physical, chemical or biochemical change in the surroundings which is induced by the intraoral microorganisms.

7. Dental material according to any one of Claims 1 to 6, **characterized in that** the substance is released owing to enzymatic detachment.

8. Dental material according to any one of Claims 1 to 4, **characterized in that** the substance is prevented from diffusing out of the dental material by derivatization, or is covalently bonded in the dental material and is stored in the region between dentine or enamel and dental material at the surface of the dental material and the development of activity is based on the substance being modified by an enzymatic, physical, chemical or biochemical change in the surroundings which is induced by the intraoral microorganisms, wherein no release of the substance takes place.

9. Dental material according to Claim 8, **characterized in that** the development of activity is brought about in a plurality of steps by a similar or different enzymatic, physical, chemical or biochemical change in the surroundings which is induced by the intraoral microorganisms.

10. Dental material according to Claim 8 or 9, **characterized in that** the substance after development of activity remains prevented from diffusing out of the dental material by derivatization or remains covalently bonded in the dental material.

11. Dental material according to any one of Claims 1 to 10, comprising
(a) 0.01 - 10% of a substance whose bacteriostatic and/or bactericidal activity is developed in the presence of intraoral microorganisms,
(b) 3 - 80% of a polymerizable compound
(c) 0.01 - 25% of customary initiators and/or accelerants and/or retarders
(d) 0 - 50% of customary excipients
(e) 0 - 90% of customary fillers.

12. Dental material according to any one of Claims 1 to 11, comprising
(a) 0.1 - 5% of a substance whose bacteriostatic and/or bactericidal activity is developed in the presence of intraoral microorganisms,
(b) 3 - 80% of a polymerizable compound
(c) 0.01 - 25% of customary initiators and/or accelerants and/or retarders
(d) 0 - 50% of customary excipients
(e) 0 - 90% of customary fillers.

13. Dental material according to any one of Claims 1 to 12, comprising
(a) 0.1 - 3% of a substance whose bacteriostatic and/or bactericidal activity is developed in the presence of intraoral microorganisms,
(b) 3 - 80% of a polymerizable compound
(c) 0.01 - 25% of customary initiators and/or accelerants and/or retarders
(d) 0 - 50% of customary excipients
(e) 0 - 90% of customary fillers.

14. Use of a substance whose bacteriostatic and/or bactericidal activity is developed in the presence of intraoral microorganisms in the manufacture of a dental material, wherein this substance comprises taurolidine and the dental materials are selected from a dental moulding material, a dental filling material, a glass ionomer cement or a dental provisional filling material.

15. Use of a substance whose bacteriostatic and/or bactericidal activity is developed in the presence of intraoral microorganisms in the manufacture of a dental moulding material, a dental filling material, a glass ionomer cement or a dental provisional filling material, wherein this substance comprises taurolidine.

## Revendications

1. Matériau dentaire contenant au moins une substance dont l'activité bactériostatique et/ou bactéricide est développée en présence de microorganismes intra-oraux, notamment la taurolidine, le matériau dentaire étant choisi parmi une masse de moulage dentaire, un matériau de remplissage dentaire, un ciment ionomère de verre ou un matériau de remplissage dentaire provisoire.

2. Matériau dentaire selon la revendication 1, **caractérisé en ce que** le développement de l'activité repose sur une modification de la substance qui est provoquée par une modification enzymatique, physique, chimique ou biochimique du milieu, déclenchée par les microorganismes infra-oraux.

3. Matériau dentaire selon l'une quelconque des revendications 1 ou 2, **caractérisé en ce que** la substance est enrichie et/ou mise à disposition dans la zone entre la dentine ou l'émail et le matériau dentaire.

4. Matériau dentaire selon l'une quelconque des revendications 1 à 3, **caractérisé en ce que** la substance s'enrichit par diffusion dans la zone entre la dentine ou l'émail et le matériau dentaire.

5. Matériau dentaire selon l'une quelconque des revendications 1 à 3, **caractérisé en ce que** la substance est empêchée de diffuser hors du matériau dentaire par dérivation ou est liée par covalence dans le matériau dentaire et est mise à disposition dans la zone entre la dentine ou l'émail et le matériau dentaire à la surface du matériau dentaire et **en ce que** la substance est libérée spécifiquement dans un lieu et dans le temps en raison de la modification enzymatique, physique, chimique ou biochimique du milieu, déclenchée par les microorganismes intra-oraux.

6. Matériau dentaire selon la revendication 5, **caractérisé en ce que** la libération spécifique dans un lieu et dans le temps de la substance et le développement de l'activité sont provoqués par une modification enzymatique, physique, chimique ou biochimique identique ou différente du milieu, déclenchée par les microorganismes intra-oraux,

7. Matériau dentaire selon l'une quelconque des revendications 1 à 6, **caractérisé en ce que** la libération de la substance est réalisée en raison d'une dissociation enzymatique.

8. Matériau dentaire selon l'une quelconque des revendications 1 à 4, **caractérisé en ce que** la substance est empêchée de diffuser hors du matériau dentaire par dérivation ou est liée par covalence dans le matériau dentaire et est mise à disposition dans la zone entre la dentine ou l'émail et le matériau dentaire à la surface du matériau dentaire et le développement de l'activité repose sur une modification de la substance active qui est provoquée par une modification enzymatique, physique, chimique ou biochimique du milieu, déclenchée par les microorganismes intra-oraux, où il ne se produit pas de libération de la substance.

9. Matériau dentaire selon la revendication 8, **caractérisé en ce que** le développement de l'activité est provoqué en plusieurs étapes par une modification enzymatique, physique, chimique ou biochimique identique ou différente du milieu, déclenchée par les microorganismes intra-oraux.

10. Matériau dentaire selon la revendication 8 ou 9, **caractérisé en ce qu'**après le développement de l'activité la substance continue à être empêchée de diffuser hors du matériau dentaire par dérivation ou reste liée par covalence dans le matériau dentaire.

11. Matériau dentaire selon l'une quelconque des revendications 1 à 10, contenant
(a) 0,01 à 10% d'une substance dont l'activité bactériostatique et/ou bactéricide est développée en présence de microorganismes intra-oraux,
(b) 3 à 80% d'un composé polymérisable
(c) 0,01 à 25% d'initiateurs et/ou d'accélérateurs et/ou de retardateurs usuels
(d) 0 à 50% d'adjuvants usuels
(e) 0 à 90% de charges usuelles.

12. Matériau dentaire selon l'une quelconque des revendications 1 à 11, contenant
(a) 0,1 à 5% d'une substance dont l'activité bactériostatique et/ou bactéricide est développée en présence de microorganismes intra-oraux,
(b) 3 à 80% d'un composé polymérisable
(c) 0,01 à 25% d'initiateurs et/ou d'accélérateurs et/ou de retardateurs usuels
(d) 0 à 50% d'adjuvants usuels
(e) 0 à 90% de charges usuelles.

13. Matériau dentaire selon l'une quelconque des revendications 1 à 12, contenant
(a) 0,1 à 3% d'une substance dont l'activité bactériostatique et/ou bactéricide est développée en présence de microorganismes intra-oraux,
(b) 3 à 80% d'un composé polymérisable
(c) 0,01 à 25% d'initiateurs et/ou d'accélérateurs et/ou de retardateurs usuels
(d) 0 à 50% d'adjuvants usuels
(e) 0 à 90% de charges usuelles.

14. Utilisation d'une substance dont l'activité bactériostatique et/ou bactéricide est développée en présence de microorganismes intra-oraux pour la préparation d' un matériau dentaire, où il s'agit, pour cette substance, de taurolidine et où les matériaux dentaires sont choisis parmi une masse de moulage dentaire, un matériau de remplissage dentaire, un ciment ionomère de verre ou un matériau de remplissage dentaire provisoire.

15. Utilisation d'une substance dont l'activité bactériostatique et/ou bactéricide est développée en présence de microorganismes infra-oraux pour la préparation d'une masse de moulage dentaire, d'un matériau de remplissage dentaire, d'un ciment ionomère de verre ou d'un matériau de remplissage dentaire provisoire, où il s'agit, pour cette substance, de taurolidine.
